# EUROPÄISCHE PATENTSCHRIFT

(11) **EP 2 774 576 B1**
(45) Veröffentlichungstag und Bekanntmachung des Hinweises auf die Patenterteilung: **13.01.2016**
(21) Anmeldenummer: 14450009.7
(22) Anmeldetag: 06.03.2014
(51) Int. Cl.: A61C 9/00, A61G 15/16

(54) **Halterung für einen Intraoralscanner**
Holder for an intra-oral scanner
Support pour un scanner intra-oral

(30) Priorität: 08.03.2013 AT 772013
(43) Veröffentlichungstag der Anmeldung: 10.09.2014
(73) Patentinhaber: a.tron3d GmbH, 9020 Klagenfurt am Wörthersee (AT)
(72) Erfinder: Koinig, Horst, 9020 Klagenfurt (AT); Jesenko, Jürgen, 9584 Finkenstein (AT); Isola, Franz, 9545 Radenthein (AT)
(74) Vertreter: Beer & Partner Patentanwälte KG

(56) Entgegenhaltungen:
- WO-A1-02/065937
- US-A1- 2012 213 572
- US-A1- 2012 251 973

## Beschreibung

Die Erfindung betrifft eine Halterung mit einem Grundkörper in Verbindung mit einem Handstück eines Intraoralscanners, welches einen Kopfbereich aufweist.

Intraoralscanner finden in der Dentalmedizin zunehmend Verwendung, da sie viele Vorteile gegenüber der herkömmlichen Erfassung der Zahnstruktur von Patienten haben. Abdrücke, die für den Patienten unangenehm sind, entfallen und strahlungsbasierte Aufnahmemethoden wie das Röntgen können zum Teil vermieden werden. Nachteilig ist allerdings der häufig erhebliche Platzbedarf, da viele Systeme als integrale Geräte erhältlich sind, bei denen Computer, Bildschirm und Handstück eine Einheit bilden. Bei diesen Ausführungsformen wird der Scanner auf einem rollbaren Wagen zum Behandlungsstuhl gefahren. Andere Systeme können mit herkömmlichen Rechnern betrieben werden. In solchen Fällen erfolgt die visuelle Ausgabe der Scans über das normale Computerdisplay. Das jedenfalls notwendige Handstück kann dann beispielsweise über einen USB-Port an den Computer angeschlossen werden. Manchmal befinden sich hierfür eigene Schnittstellen am Behandlungsstuhl. Besonders fortschrittliche Systeme verfügen über die Möglichkeit einer drahtlosen Datenübertragung und können so besonders komfortabel genutzt werden. Ein Problem stellt sich bei allen Systemen dann, wenn der Scanner nicht genutzt wird. Ein großer Wagen ist generell unpraktisch und die üblichen, an einem Behandlungsstuhl angebrachten, Aufbewahrungsmöglichkeiten sind in der Regel nicht für Intraoralscanner geeignet, da diese sich in ihrer Form in der Regel von den sonst üblichen Behandlungsgeräten unterscheiden.

Aufgabe der Erfindung ist es daher, eine gegebenenfalls nachrüstbare und für den Nutzer besonders komfortable Halterung für einen Intraoralscanner zur Verfügung zu stellen.

Gelöst wird diese Aufgabe durch eine Halterung der eingangs genannten Art, die durch einen Aufnahmebereich des Grundkörpers, in welchem der Kopfbereich wenigsten teilweise formschlüssig aufnehmbar ist und der eine Innenseite mit wenigstens zwei Haltebereichen aufweist, die einander im Wesentlichen gegenüber liegend angeordnet sind, gekennzeichnet ist.

So kann der Intraoralscanner mit seinem Kopfbereich einfach und schnell in die Halterung gehängt bzw. gelegt werden.

Weitere bevorzugte und vorteilhafte Ausführungsformen der Erfindung sind Gegenstand der Unteransprüche.

Weitere Einzelheiten und Merkmale der Erfindung sowie Vorteile derselben ergeben sich aus der nachstehenden Beschreibung bevorzugter Ausführungsformen der Erfindung unter Bezugnahme auf die in den Zeichnungen beispielhaft dargestellten Ausführungsformen. Es zeigt:
- Fig. 1: eine isometrische Ansicht einer ersten Ausführungsform der erfindungsgemäßen Halterung mit einem Scanner,
- Fig. 2: eine schematisierte Seitenansicht der Ausführungsform von Fig. 1,
- Fig. 3: eine erste isometrische Ansicht einer zweiten Ausführungsform der Halterung,
- Fig. 4: eine zweite isometrische Ansicht der zweiten Ausführungsform,
- Fig. 5: eine Ansicht der zweiten Ausführungsform mit einem Scanner von oben,
- Fig. 6: einen Längsschnitt durch die Ausführungsform von Fig. 5,
- Fig. 7: einen ersten Schnitt durch die Ausführungsform von Fig. 5 und 6,
- Fig. 8: einen zweiten Schnitt durch die Ausführungsform von Fig. 5 und 6,
- Fig. 9: eine isometrische Ansicht einer dritten Ausführungsform der Halterung,
- Fig. 10: eine Ansicht der dritten Ausführungsform von oben und
- Fig. 11: einen Schnitt durch die Fig. 10.

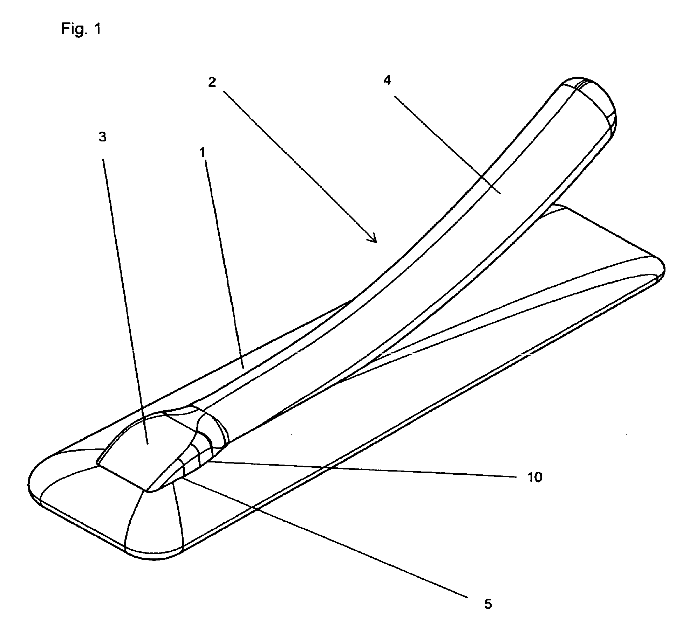

Die Fig. 1 zeigt eine isometrische Ansicht einer ersten Ausführungsform der erfindungsgemäßen Halterung mit einem Scanner 2. Ein Grundkörper 1 der Halterung nimmt den Scanner 2 im Bereich des Scannerkopfes 3 und teilweise im Bereich des Scanngergriffes 4 in einem Aufnahmebereich 5 auf.

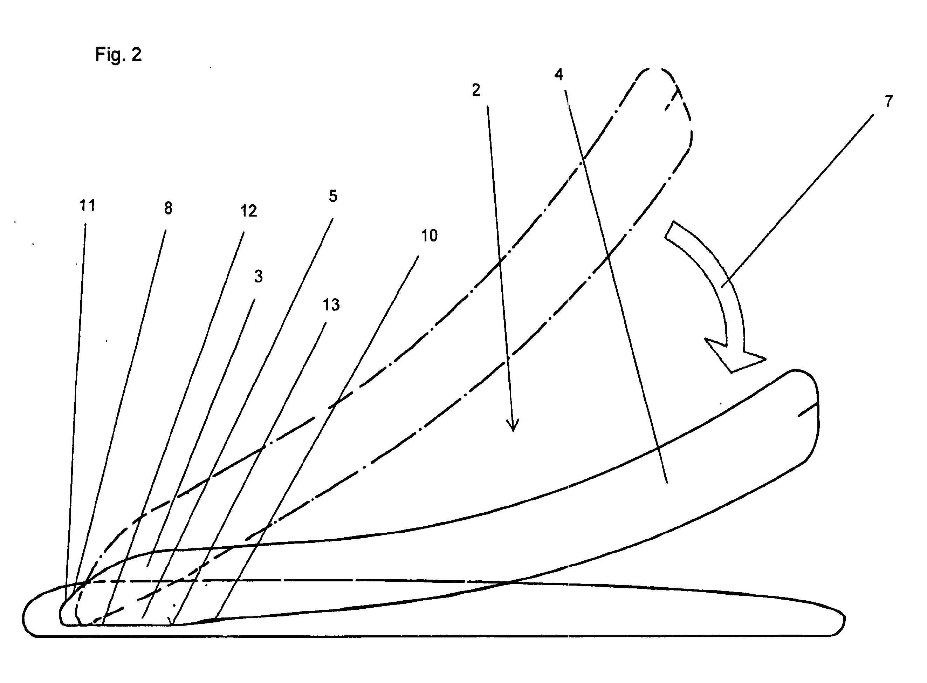

Die Fig. 2 zeigt einen schematisierten Längsschnitt durch die Ausführungsform von Fig. 1. Eine strichpunktierte Scannerumrandung 6 zeigt dabei den Beginn einer in Richtung eines Pfeils 7 erfolgenden Ablagebewegung. Diese kann einfach und intuitiv ausgeführt werden. Es sind keine komplizierten Verriegelungsmechanismen erforderlich, welche die Handhabung für den Nutzer weniger komfortabel gestalten würden.

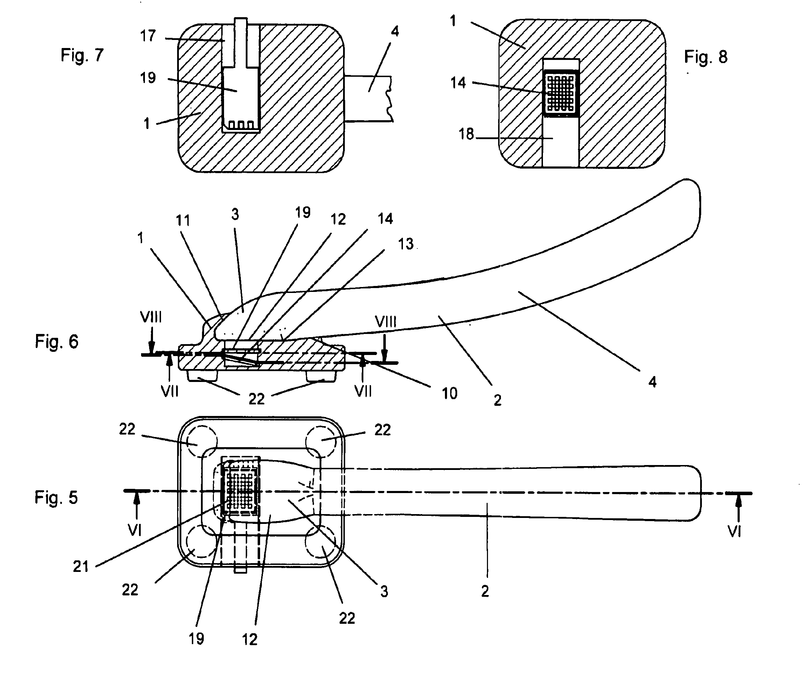

Die Fig. 2 zeigt weiters die Kontur einer Innenseite 8 des Aufnahmebereiches 5. Diese weist zwei einander im wesentlichen gegenüberliegende Haltebereiche 10, 11 auf. Der hintere Haltebereich 10 nimmt dabei das Gewicht des Scanners 2 auf und zieht sich seitlich nach oben, wie Fig. 1 zeigt. Der vordere Haltebereich 11 wirkt dabei einer Kipp-Bewegung entgegen. Außerdem kann der Scannerkopf 3 bevorzugt leicht festgeklemmt werden. So kann das Gewicht des Scannergriffes 4 besser aufgenommen werden. Zur Unterstützung des Haltes der Scanners 2 im Aufnahmebereich 5 können die Haltebereiche 10, 11 ein rutschfestes Material, beispielsweise Silikon, aufweisen oder daraus bestehen. Ein Arbeitsbereich 12 des Scanners 2 mit beispielsweise einem Scannerglas kommt dabei auf einem Grundbereich 13 der Innenseite 8 des Aufnahmebereiches 5 zu liegen und ist diesem zugeornet. Diese Grundfläche 13 kann erfindungsgemäß verschiedenen weitere vorteilhafte technische Merkmale aufweisen. So kann das Scannerglas, welches sich üblicherweise im Arbeitsbereich 13 eines Intraoralscanners befindet, beispielsweise über eine beheizte Grundfläche 13 vorgewärmt werden, was einem späteren Beschlagen der Scheibe bei der Verwendung des Scanners 2 vorbeugt. Hierfür kann beispielsweise ein Heizelement 14 (Fig. 5 bis 8) vorgesehen sein.

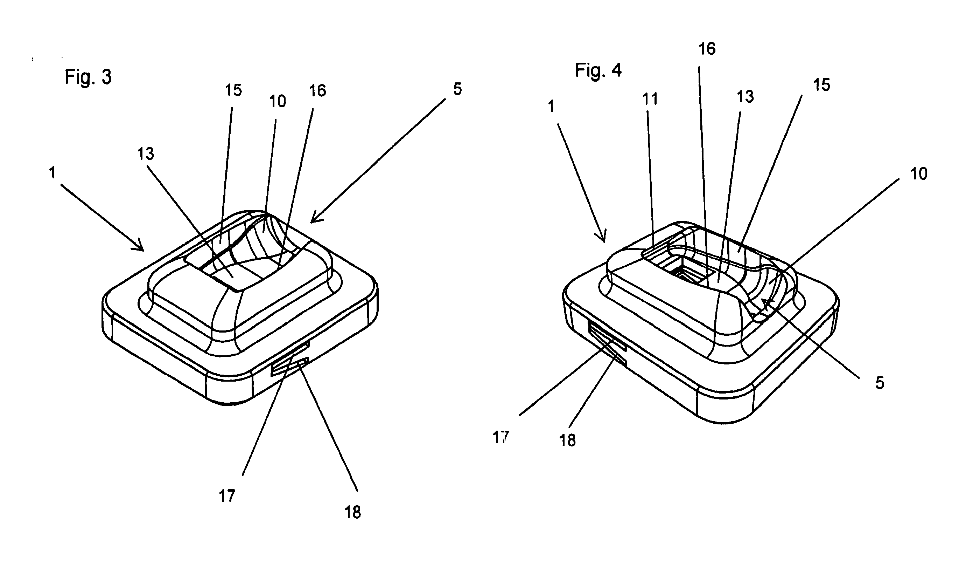

Die Fig. 3 und Fig. 4 zeigen eine erste und eine zweite isometrische Ansicht einer zweiten Ausführungsform eines Grundkörpers 1 der Halterung. Der Aufnahmebereich 5 ist dabei nicht wie in der ersten Ausführungsform im Grundkörper 1 vertieft aufgenommen, sondern erhebt sich über diesem. Der Aufnahmebereich 5 nimmt in dieser bevorzugten Ausführungsform im Wesentlichen nur den Scannerkopf 3 auf. Neben den Haltebereichen 10, 11 erkennt man Begrenzungsbereiche 15, 16, die in dieser Ausführungsform mit dem Grundbereich 13 verbunden sind. Diese Begrenzungsbereiche 15, 16 verhindern ein Abrutschen des Scanners 2 zur Seite und führen die Ablagebewegung. Die Haltebereiche 10, 11 bilden in dieser Ausführungsform mit den Begrenzungsbereichen 15, 16 eine Umrandung des Aufnahmebereiches 5.

Weiters erkennt man an einer Seite des Grundkörpers 1 Ausnehmungen 17, 18, die zur Seite des Grundkörpers hin offen sind. In diesen Ausnehmungen 17, 18 können beispielsweise Heizelemente 14 oder Kalibrierelemente 19 (Fig. 5 bis 8) aufgenommen werden. Ein Kalibrierelement 19 weist dabei ein Kalibriermuster 21 (Fig. 5) auf, welches für Kalibrierungen des Scanners genutzt werden kann. Dies können beispielsweise Flat-Field-Corrections oder auch Korrekturen von wärmebedingten Verzerrungen der Optik des Scanners sein. Dabei wird während des Lagerns des Scanners in der Halterung das bekannte Kalibriermuster 21 aufgenommen. Verzerrungen des Musters können in einem Rechner erkannt und rechnerisch korrigiert werden. Bevorzugt können dafür verschiedene Kalibrierelemente 19 in den Grundkörper gebracht werden, da für unterschiedliche Kalibrierungen unterschiedliche Kalibriermuster 21 vorteilhaft sein können. Vorteilhafterweise kann die Halterung den Arbeits- oder auch Messbereich des Scanners dabei vollständig abdunkeln, um Störungen beim Kalibrieren zu vermeiden.

Gemäß einer alternativen oder zusätzlichen Ausführungsform der Erfindung kann das Kalibrieren auch ohne ein Muster erfolgen. Dabei wird der bekannte Abstand zwischen dem Scannerkopf und dem Kalibrierelement vermessen und der gemessene und der reale Wert miteinander verglichen. Entsprechend der Abweichung können dann Korrekturen bzw. Kalibrierungen vorgenommen werden. In einer Weiterbildung der Erfindung, die auch bei Kalibrierelementen mit Kalibriermuster Anwendung finden kann, ist das Kalibrierelement um einen bekannten Winkel zum Arbeitsbereich geneigt. So stehen mehrere bekannte Abstände für Kalibrierungen zur Verfügung, und das Kalibrieren kann noch genauer erfolgen. In einer weiteren Weiterbildung ist der Winkel bzw. die Position des Kalibrierelementes zum Arbeitsbereich, bzw. der Grundfläche, beispielsweise über einen Schalter oder Hebel, verstellbar. Dies kann gegebenfalls mit einer Verstelleinrichtung für das Heizelement kombiniert werden; so kann beispielsweise zunächst kalibriert werden und dann das Heizelement an den Arbeitsbereich geschoben werden, um diesen zu wärmen. Das Muster kann auch vom Scanner selbst auf die Oberfläche des Kalibrierelementes projiziert werden. Dann muss das Muster nicht dauerhaft auf dem Kalibrierelement angebracht sein.

In einer Weiterbildung der Erfindung ist es alternativ oder zusätzlich auch möglich, dass das Kalibrierelement ein Objekt mit einer bekannten, gegebenenfalls komplexeren, unebenen Oberflächenstruktur ist. Das Objekt kann sich optional auch bewegen, beispielsweise drehen. Der Grundkörper gestaltet sich dann entsprechend größer um das Objekt und mögliche Antriebe und Halterungen zum Bewegen des Objektes aufzunehmen.

Die Fig. 5 zeigt eine schematisierte Ansicht der zweiten Ausführungsform von Fig. 3 und 4 mit einem Scanner 2 von oben. Man erkennt unterhalb des Arbeitsbereiches 12 des Scannerkopfes 3 das Kalibrierelement 19 mit dem Kalibriermuster 21. Ausführungsformen, bei denen das Heizelement und das Kalibrierelement einteilig ausgeführt sind, beispielsweise durch ein mit einem Kalibriermuster bedrucktes Heizelement, sind ebenso denkbar. Unter dem Grundkörper 1 kann man Füße 22 erkennen. Diese können als Unterlage für den Grundkörper 1 dienen oder auch kraft- oder stoffschlüssig, beispielsweise über Saugnäpfe oder auch Klebeverbindungen, mit einem Untergrund, beispielsweise dem Behandlungsstuhl oder einer Tischoberfläche, verbunden sein.

Die Fig. 6 zeigt einen Längsschnitt durch die Fig. 5. Man erkennt die Position des Kalibrierelementes 19 und des Heizelementes 14 im Grundkörper 1. Der dem Grundbereich 13 zugeordnete Arbeitsbereich 12 liegt dabei über dem Kalibrierelement 19. Hierzu ist in dieser Ausführungsform ein Abstand zwischen dem Kalibrierelement 19 und dem Arbeitsbereich 12 vorgesehen. Ausführungsformen, bei denen der Arbeitsbereich 12 und das Kalibriermuster 21 nicht voneinander beabstandet sind, sind ebenso denkbar.

Die Fig. 7 zeigt einen ersten Schnitt entlang der Linie VII-VII in Fig. 6. Man erkennt das Kalibrierelement 19 in der Ausnehmung 17 im Grundkörper 1. In einer Weiterbildung der Erfindung kann das Kalibrierelement 19 auch weitere Aufgaben übernehmen und beispielweise eine drahtlose Datenverbindung zum Scanner herstellen und gegebenenfalls Daten, welche zuvor vom Scanner erfasst wurden, auslesen. Natürlich kann ein Mittel zur Datenübertragung, gegebenenfalls auch über eine galvanische Verbindung, gesondert im Grundkörper angeordnet sein.

Die Fig. 8 zeigt einen zweiten Schnitt entlang der Linie VIII-VIII in Fig. 6. Man erkennt das Heizelement 14 in der Ausnehmung 18 im Grundkörper 1.

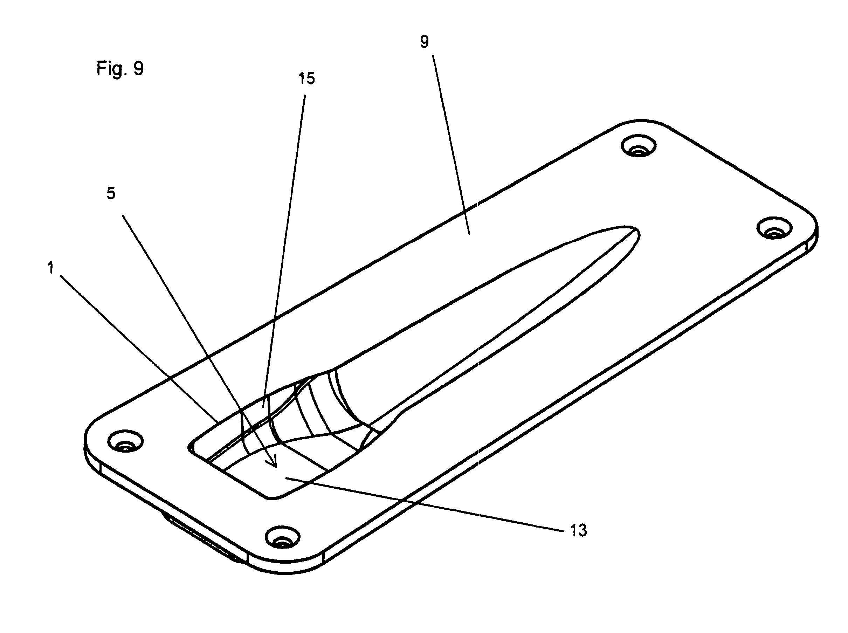

Die Fig. 9 zeigt eine isometrische Ansicht einer dritten Ausführungsform der Halterung. Bei dieser wird der Grundkörper 1 nicht, wie bei den vorstehenden Ausführungsformen der Erfindung beschrieben, auf einer Oberfläche angeordnet, sondern in eine Oberfläche beispielsweise eines Tisches oder Behandlungsstuhles eingelassen und weist eine Montageplatte 9 auf. Der Aufnahmebereich 5 ist dabei in der Oberfläche versenkt.

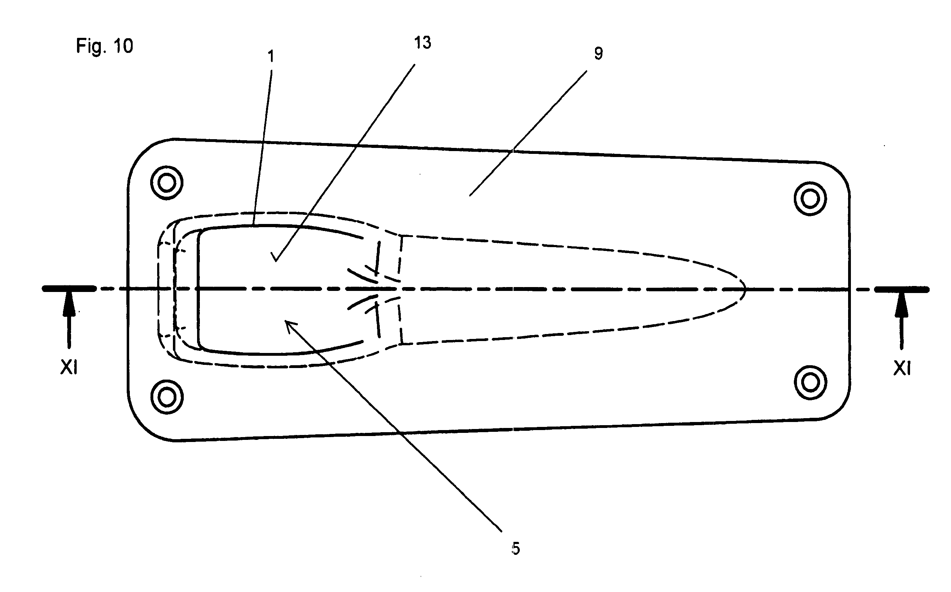

Die Fig. 10 zeigt eine Ansicht der dritten Ausführungsform von oben. Im Grundbereich 13 können auch bei dieser Ausführungsform nicht dargestellte Heiz- oder Kalibrierelemente untergebracht sein. Ein Austausch könnte dann von oben vor dem Ablegen des Scanners 2 erfolgen.

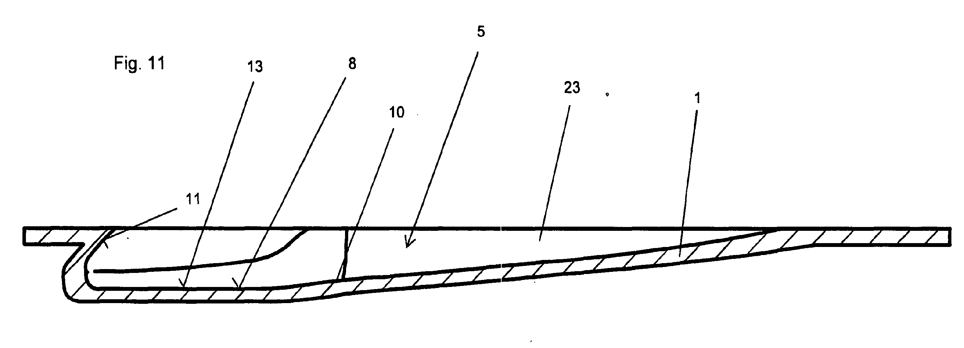

Die Fig. 11 zeigt einen Schnitt entlang der Linie XI-XI in Fig. 10. Man erkennt, wie der dem Haltebereich 10 gegenüberliegende Haltebereich 11 im Wesentlichen V-förmig zum Grundbereich 13 hin offen angeordnet ist. Der dadurch umfasste Teil der Innenseite 8 nimmt den vorderen Teil des Scannerkopfes 2 auf. Der in dieser Ausführungsform besonders lange, hintere Bereich 23 des Aufnahmebereiches 5 kann in einer Weiterbildung der Erfindung für weitere Funktionen der Halterung genutzt werden. So kann im Grundkörper 1 beispielsweise ein Ladegerät angeordnet sein, das induktiv oder über eine galvanische Verbindung zum Laden eines Akkus im Scanner 2 dient.

Ein drahtloser Scanner könnte so zum Beispiel während der Ablage in der Halterung Daten austauschen und sich kalibrieren. Außerdem kann der Scannerkopf für eine folgende Behandlung vorgewärmt und der Scanner gleichzeitig geladen werden. Um eine oder mehrere dieser Funktionen in Gang zu setzten, kann ein Schalter am Grundkörper vorgesehen sein. Dieser wird in einer bevorzugten Weiterbildung der Erfindung beim Einsetzen bzw. Herausnehmen des Scanners geschaltet. Heizung, Ladefunktion usw. werden also erst dann bestromt, wenn sich auch tatsächlich ein Scanner in der Halterung befindet. Dies spart Energie und beugt möglichen Beschädigungen, beispielweise durch ein Überhitzen der Halterung bzw. des Heizelementes, vor.

In einer Weiterbildung der Erfindung kann zusätzlich auch ein Sensor für die Temperatur der Außenseite des Scanner vorgesehen sein. Dieser kann seine Informationen beispielsweise über ein Display an der Halterung anzeigen oder auch an einen mit dem Scanner und/oder der Halterung verbunden Rechner übermitteln. In einer speziellen Anwendung der Halterung kann auch die Temperatur des Heizelementes aufgrund der Sensorinformationen geregelt werden. Dies kann beispielsweise dann sinnvoll sein, wenn der Scanner in der Tierzahnheilkunde eingesetzt werden soll, da die Temperatur des Arbeitsbereiches der Körpertemperatur der jeweiligen Tierart angepasst werden kann.

## Patentansprüche

1. Halterung mit einem Grundkörper in Verbindung mit einem Handstück eines Intraoralscanners, welches einen Kopfbereich (3) aufweist, **gekennzeichnet durch** einen Aufnahmebereich (5) des Grundkörpers (1), in welchem der Kopfbereich (3) wenigsten teilweise formschlüssig aufnehmbar ist und der eine Innenseite (8) mit wenigstens zwei Haltebereichen (10, 11) aufweist, die einander im Wesentlichen gegenüber liegend angeordnet sind.

2. Halterung nach Anspruch 1, **dadurch gekennzeichnet, dass** die Innenseite (8) einen Grundbereich (13) aufweist, der einem Arbeitsbereich (12) des Kopfbereiches (3) zugeordnet ist.

3. Halterung nach Anspruch 2, **dadurch gekennzeichnet dass** wenigstens ein Haltebereich (11) eine U- oder V-förmige Halterung ist, die zum Aufnahmebereich (5) offen ist.

4. Halterung nach einem der Ansprüche 1 bis 3, **dadurch gekennzeichnet, dass** die Innenseite (8) wenigstens einen, vorzugsweise zwei, Begrenzungsbereiche (15, 16) für den Aufnahmebereich (5) aufweist.

5. Halterung nach Anspruch 4, **dadurch gekennzeichnet, dass** der Begrenzungsbereich (15, 16) an den Grundbereich (13) angrenzt.

6. Halterung nach Anspruch 4 oder 5, **dadurch gekennzeichnet, dass** der Begrenzungsbereich (15, 16) wenigstens teilweise Haltebereiche (10, 11) beinhaltet.

7. Halterung nach einem der Ansprüche 1 bis 6, **dadurch gekennzeichnet, dass** der Grundbereich (13) ein beheizbares elektrisches oder elektronisches Heizelement (14) aufweist.

8. Halterung nach einem der Ansprüche 1 bis 7, **dadurch gekennzeichnet, dass** der Grundkörper (1) ein induktives Ladegerät aufweist.

9. Halterung nach einem der Ansprüche 1 bis 7, **dadurch gekennzeichnet, dass** der Grundkörper (1) galvanische Kontakte eines Ladegerätes aufweist.

10. Halterung nach einem der Ansprüche 2 bis 9, **dadurch gekennzeichnet, dass** der Grundbereich (13) ein Kalibriermuster (21) aufweist.

11. Halterung nach Anspruch 10, **dadurch gekennzeichnet, dass** das Heizelement (14) das Kalibriermuster (21) aufweist.

12. Halterung nach Anspruch 10, **dadurch gekennzeichnet, dass** das Heizelement (14) und das Kalibriermuster (21) nebeneinander angeordnet sind.

13. Halterung nach einem der Ansprüche 10 bis 12, **dadurch gekennzeichnet, dass** der Grundkörper (1) eine Ausnehmung (17, 18) aufweist, in der ein Heizelement (14) oder ein Kalibrierelement (19) aufnehmbar sind.

14. Halterung nach Anspruch 13, **dadurch gekennzeichnet, dass** das Kalibrierelement (19) ein Kalibriermuster (21) aufweist.

15. Halterung nach Anspruch 13 oder 14, **dadurch gekennzeichnet, dass** die Position(en) des Heizelementes (14) und/oder des Kalibrierelementes (19) über einen Schalter oder Hebel verstellbar ist/sind.

16. Halterung nach einem der Ansprüche 13 bis 15, **dadurch gekennzeichnet, dass** die Ausnehmung (17, 18) zu einer äußeren Seite des Grundkörpers (1) hin geöffnet ist.

17. Halterung nach einem der Ansprüche 7 bis 16, **dadurch gekennzeichnet, dass** der Grundkörper (1) wenigstens einen Schalter aufweist, mit dem das Heizelment (14) und/oder das Ladegerät eingeschalten werden können.

18. Halterung nach einem der Ansprüche 1 bis 17, **dadurch gekennzeichnet, dass** der Grundkörper (1) einen Auflageschalter aufweist, dessen Schalterstellung durch die An- bzw. Abwesenheit des Scanners bzw. Scannerkopfes gesteuert ist.

19. Halterung nach Anspruch 18, **dadurch gekennzeichnet, dass** der Auflageschalter das Heizelement (14) und/oder das Ladegerät ein- bzw. ausschaltet.

20. Halterung nach einem der Ansprüche 1 bis 19, **dadurch gekennzeichnet, dass** der Grundkörper (1) ein Mittel zur Datenübertragung zwischen dem Grundkörper (1) und dem Handstück aufweist.

21. Halterung nach einem der Ansprüche 1 bis 20, **dadurch gekennzeichnet, dass** der Grundkörper (1) einen Messbereich des Handstücks vollständig abdeckt.

22. Halterung nach einem der Ansprüche 1 bis 21, **dadurch gekennzeichnet, dass** die Halterung einen Temperatursensor aufweist.

23. Halterung nach Anspruch 22, **dadurch gekennzeichnet, dass** die Halterung eine Anzeige, insbesondere Display, für einen vom Temperatursensor ermittelten Wert aufweist.

## Claims

1. Mounting having a base body in conjunction with a handpiece of an intraoral scanner, which has a head region (3), **characterized by** a accommodation region (5) of the base body (1), in which the head region (3) can be accommodated at in an least partially form-fitting manner, and which has an inner surface (8) having at least two holding regions (10, 11) arranged essentially opposite to one another.

2. Mounting according to claim 1, **characterized in that** the inner surface (8) has a base region (13), which is assigned to a working region (12) of the head region (3).

3. Mounting according to claim 2, **characterized in that** at least one holding region (11) is a U-shaped or V-shaped mounting, which is open to the accommodation region (5).

4. Mounting according to any one of claims 1 to 3, **characterized in that** the inner surface (8) has at least one, preferably two, bordering regions (15, 16) for the accommodation region (5).

5. Mounting according to claim 4, **characterized in that** the bordering region (15, 16) is adjacent to the base region (13).

6. Mounting according to claim 4 or 5, **characterized in that** the bordering region (15, 16) contains at least partial holding regions (10, 11).

7. Mounting according to any one of claims 1 to 6, **characterized in that** the base region (13) has a heatable electric or electronic heating element (14).

8. Mounting according to any one of claims 1 to 7, **characterized in that** the base body (1) has an inductive charging device.

9. Mounting according to any one of claims 1 to 7, **characterized in that** the base body (1) has galvanic contacts of a charging device.

10. Mounting according to any one of claims 2 to 9, **characterized in that** the base region (13) has a calibration pattern (21).

11. Mounting according to claim 10, **characterized in that** the heating element (14) has the calibration pattern (21).

12. Mounting according to claim 10, **characterized in that** the heating element (14) and the calibration pattern (21) are arranged side by side.

13. Mounting according to any one of claims 10 to 12, **characterized in that** the base body (1) has a recess (17, 18), in which a heating element (14) or a calibration element (19) can be accommodated.

14. Mounting according to claim 13, **characterized in that** the calibration element (19) has a calibration pattern (21).

15. Mounting according to claim 13 or 14, **characterized in that** the position(s) of the heating element (14) and/or of the calibration element (19) is/are adjustable by means of a switch or a lever.

16. Mounting according to any one of claims 13 to 15, **characterized in that** the recess (17, 18) is open toward an exterior side of the base body (1).

17. Mounting according to any one of claims 7 to 16, **characterized in that** the base body (1) has at least one switch with which the heating element (14) and/or the charging device can be turned on.

18. Mounting according to any one of claims 1 to 17, **characterized in that** the base body (1) has a contact switch, whose switch position is controlled by the presence or absence of the scanner or the scanner head, respectively.

19. Mounting according to claim 18, **characterized in that** the contact switch switches the heating element (14) and/or the charging device on or off, respectively.

20. Mounting according to any one of claims 1 to 19, **characterized in that** the base body (1) has a means for data transmission between the base body (1) and the handpiece.

21. Mounting according to any one of claims 1 to 20, **characterized in that** the base body (1) completely covers a measurement range of the handpiece.

22. Mounting according to any one of claims 1 to 21, **characterized in that** the mounting has a temperature sensor.

23. Mounting according to claim 22, **characterized in that** the mounting has a display, in particular a display screen, for a value determined by the temperature sensor.

## Revendications

1. Fixation comprenant un corps de base en relation à une pièce à main d'un scanner intraoral, laquelle présente une zone de tête (3), **caractérisée par** une zone de réception (5) du corps de base (1) dans laquelle la zone de tête (3) peut être reçue au moins partiellement par complémentarité de forme et qui présente une face intérieure (8) comprenant au moins deux zones de fixation (10, 11) qui sont disposées en étant essentiellement opposées l'une à l'autre.

2. Fixation selon la revendication 1, **caractérisée en ce que** la face intérieure (8) présente une zone de base (13) qui est attribuée à une zone de travail (12) de la zone de tête (3).

3. Fixation selon la revendication 2, **caractérisée en ce qu'**au moins une zone de fixation (11) est une fixation en forme de U ou en forme de V qui est ouverte en direction de la zone de réception (5).

4. Fixation selon l'une des revendications 1 à 3, **caractérisée en ce que** la face intérieure (8) présente au moins une, de préférence deux, zone(s) de délimitation (15, 16) pour la zone de réception (5).

5. Fixation selon la revendication 4, **caractérisée en ce que** la zone de délimitation (15, 16) est délimitée sur la zone de base (13).

6. Fixation selon la revendication 4 ou 5, **caractérisée en ce que** la zone de délimitation (15, 16) contient des zones de fixation (10, 11), au moins partiellement.

7. Fixation selon l'une des revendications 1 à 6, **caractérisée en ce que** la zone de fond (13) présente un élément chauffant (14) électrique ou électronique pouvant être chauffé.

8. Fixation selon l'une des revendications 1 à 7, **caractérisée en ce que** le corps de base (1) est un chargeur inductif.

9. Fixation selon l'une des revendications 1 à 7, **caractérisée en ce que** le corps de base (1) présente des contacts galvaniques d'un chargeur.

10. Fixation selon l'une des revendications 2 à 9, **caractérisée en ce que** la zone de fond (13) présente un motif de calibrage (21).

11. Fixation selon la revendication 10, **caractérisée en ce que** l'élément chauffant (14) présente le motif de calibrage (21).

12. Fixation selon la revendication 10, **caractérisée en ce que** l'élément chauffant (14) et le motif de calibrage (21) sont disposés l'un à côté de l'autre.

13. Fixation selon l'une des revendications 10 à 12, **caractérisée en ce que** le corps de base (1) présente un évidement (17, 18) dans lequel un élément chauffant (14) ou un élément de calibrage (19) peuvent être reçus.

14. Fixation selon la revendication 13, **caractérisé en ce que** l'élément de calibrage (19) présente un motif de calibrage (21).

15. Fixation selon la revendication 13 ou 14, **caractérisé en ce que** la/les position(s) de l'élément chauffant (14) et/ou de l'élément de calibrage (19) peu(ven)t être réglée(s) par un commutateur ou un levier.

16. Fixation selon l'une des revendications 13 à 15, **caractérisée en ce que** l'évidement (17, 18) est ouvert en direction d'un côté extérieur du corps de base (1).

17. Fixation selon l'une des revendications 7 à 16, **caractérisée en ce que** le corps de base (1) comprend au moins un commutateur avec lequel l'élément chauffant (14) et/ou le chargeur peu(ven)t être mis en marche.

18. Fixation selon l'une des revendications 1 à 17, **caractérisée en ce que** le corps de base (1) présente un commutateur à support dont la position de commutateur est commandée par la présence, respectivement l'absence du scanner, respectivement de la tête de scanner.

19. Fixation selon la revendication 18, **caractérisé en ce que** le commutateur d'appui met en marche, respectivement arrête, l'élément chauffant (14) et/ou le chargeur.

20. Fixation selon l'une des revendications 1 à 19, **caractérisée en ce que** le corps de base (1) présente un moyen pour transmettre des données entre le corps de base (1) et la pièce à main.

21. Fixation selon l'une des revendications 1 à 20, **caractérisée en ce que** le corps de base (1) recouvre entièrement une zone de mesure de la pièce à main.

22. Fixation selon l'une des revendications 1 à 21, **caractérisée en ce que** la fixation présente un capteur de température.

23. Fixation selon la revendication 22, **caractérisée en ce que** la fixation présente un affichage, en particulier un écran, pour une valeur captée par le capteur de température.
